# EUROPÄISCHE PATENTSCHRIFT

(11) **EP 2 155 679 B1**
(45) Veröffentlichungstag und Bekanntmachung des Hinweises auf die Patenterteilung: **17.02.2016**
(21) Anmeldenummer: 08760145.6
(22) Anmeldetag: 28.05.2008
(51) Int. Cl.: C07D 211/58

(54) **VERFAHREN ZUR HERSTELLUNG VON 4-FORMYLAMINOPIPERIDINDERIVATEN**
METHOD FOR THE PRODUCTION OF 4-FORMYLAMINOPIPERIDINE DERIVATIVES
PROCÉDÉ DE PRÉPARATION DE DÉRIVES DE 4-FORMYLAMINOPIPERIDINE

(30) Priorität: 31.05.2007 EP 07109320
(43) Veröffentlichungstag der Anmeldung: 24.02.2010
(73) Patentinhaber: BASF SE, 67056 Ludwigshafen (DE)
(72) Erfinder: HAREMZA, Sylke, 69151 Neckargemünd (DE); BERG, Thomas, 67071 Ludwigshafen (DE)
(86) Internationale Anmeldenummer: PCT/EP2008/056550
(87) Internationale Veröffentlichungsnummer: WO 2008/145673

(56) Entgegenhaltungen:
- EP-A- 0 316 582
- US-A- 3 347 916
- TAKAHASHI, KYOKO ET AL: "Formylation of amines by dimethylformamide in the presence of hydrous zirconium oxide" AGRICULTURAL AND BIOLOGICAL CHEMISTRY , 52(3), 853-4 CODEN: ABCHA6; ISSN: 0002-1369, 1988, XP002512554

## Beschreibung

Die vorliegende Erfindung betrifft Verfahren zur Herstellung von 4-Formylaminopiperidinderivaten. Des weiteren betrifft die Erfindung Verfahren zur Isolierung und Aufarbeitung von 4-Formylaminopiperidinderivaten. Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils konkret angegebenen Kombination, sondern auch in anderen Kombinationen verwendbar sind, ohne den Rahmen der Erfindung zu verlassen.

Es ist bekannt, dass 4-Formylaminopiperidinderivate (4-N-formylierte 4-Aminopiperidin-derivate) durch Reaktion von 4-Aminopiperidinderivaten mit Ameisensäure oder Ameisensäureestern hergestellt werden. EP 0 316 582 A1 offenbart insbesondere die Verwendung von Methyl- oder Ethylestern der Ameisensäure. Entsprechend EP 0 316 582 A1 kann bei den Verfahren mit oder ohne Katalysator gearbeitet werden. Als Katalysatoren werden in EP 0 316 582 A1 Lewissäuren, insbesondere Titanorthoester und speziell Titanorthobutylat genannt.

4-Formylaminopiperidinderivate, wie beispielsweise 4-Amino-2,2,6,6-tetraalkylpiperidinderivate werden, wie in EP 0 316 582 A1 beschrieben, als Lichtschutzmittel für Polymere eingesetzt.

In US 4,789,757-A wird die N-Formylierung von Aminosäuren offenbart. Die Formylierung erfolgt durch das Erhitzen einer Suspension der Aminocarbonsäure in Formamid bei Temperaturen von 50 bis 100°C. Das Verfahren zur N-Formylierung wird in einer inerten Atmosphäre unter hohem Überschuß von Formamid bei Umsätzen von 80-100% durchgeführt.

Weiterhin ist aus DD 230527 A die Formylierung von Anilin durch Formamid in Gegenwart von Ameisensäure oder Ameisensäuresalzen bekannt. Das Formanilid wird mit Umsätzen von mehr als 70% erhalten.

US 3,347,916 beschreibt die Herstellung von N-Formylverbindungen. Die Formylierung von primären oder sekundären, alipahatischen oder aromatischen, Aminen erfolgt durch Umsetzung des Amins mit Formamid. Als Katalysator wird 10 Gew.-% Borsäure bezogen auf Formamid eingesetzt. Die Umsätze sind mit 25-26 % bezogen auf Formamid relativ gering und das entstehende Formylierungsprodukt muss aufwendig aufgereinigt werden.

Es besteht der ständige Bedarf nach verbesserten Herstellungsverfahren für 4-Formylaminopiperidinderivate, die eine einfache Reaktionsführung gestatten und hohe Umsätze ermöglichen. Weiterhin besteht der Bedarf nach Verfahren, mit denen möglichst reine 4-Formylaminopiperidinderivate zugänglich werden.

Aufgabe der vorliegenden Erfindung war es daher, ein Verfahren für die Herstellung von 4-Formylaminopiperidinderivaten zu entwickeln. Eine weitere Aufgabe war es die Herstellung von 4-Formylaminopiperidinderivaten ohne den Einsatz von potentiell korrosiven Substanzen wie Ameisensäure zu ermöglichen. Eine Teilaufgabe war es ein Verfahren mit einfacher Reaktionsführung und hohen Umsätzen zu finden. Eine weitere Aufgabe der Erfindung war es ein Verfahren zu finden, dass die Herstellung möglichst reiner Reaktionsprodukte ermöglicht.

Diese und andere Aufgaben werden, wie aus dem Offenbarungsgehalt der vorliegenden Erfindung ersichtlich, durch die verschiedenen Ausführungsformen des erfindungsgemäßen Verfahrens, die im weiteren beschrieben sind, gelöst.

Dementsprechend wurde ein Verfahren zur Herstellung von 4-Formylaminopiperidinderivaten der Formel (I) 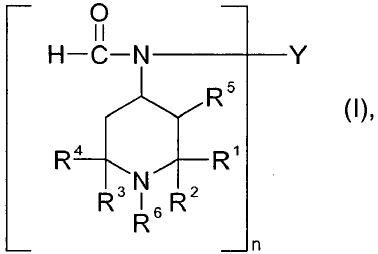 in der
n 1 oder 2,
R¹, R², R³ und R⁴ unabhängig voneinander, gleich oder verschieden, C₁-C₄-Alkyl oder R¹ und R² oder R³ und R⁴ zusammen eine Tetramethylen- oder Pentamethylengruppe,
R⁵ Wasserstoff oder C₁-C₄-Alkyl,
R⁶ Wasserstoff, Sauerstoff, C₁-C₂₂-Alkyl, C₃-C₂₂-Alkenyl, C₁-C₂₂-Alkoxy, unsubstituiertes oder substituiertes C₇-C₁₂-Phenylalkyl, C₁-C₂₂-Alkanoyl, C₂-C₃-Cyanalkyl, C₁-C₂₂-Hydroxyalkyl oder C₂-C₂₂-Aminoalkyl und
- wenn n = 1 ist -
Y Wasserstoff, C₁-C₂₂-Alkyl, C₃-C₂₂-Alkenyl, C₃-C₁₂-Cyloalkyl oder Bicycloalkyl, durch Cyan, Hydroxy oder C₁-C₄-alkoxy substituiertes C₂-C₂₂-Alkyl, durch Ethersauerstoff, Stickstoff oder Schwefel unterbrochenes C₄-C₂₂-Alkyl, unsubstituiertes oder substituiertes C₇-C₂₂-Phenyl- oder Diphenylalkyl, unsubstituiertes oder substituiertes Phenyl, ein Rest der Formel 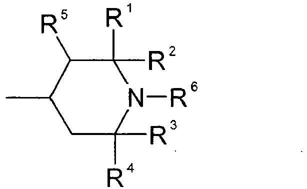 oder heterocyclische Reste enthaltendes C₁-C₂₂-Alkyl oder
- wenn n = 2 ist -
Y C₂-C₂₂-Alkylen, C₅-C₂₂-Cycloalkylen, C₈-C₁₄-Phenylalkylen, Phenylen oder durch Ethersauerstoff, Stickstoff, Schwefel oder 5 - oder 6-gliedrige Heterocyclen unterbrochenes C₄-C₃₀-Alkylen
bedeuten,
sowie den Säureadditionssalzen dieser Verbindungen,
gefunden, wobei 4-Aminopiperidinderivate der Formel (II) oder die Säureadditionssalze dieser Verbindungen 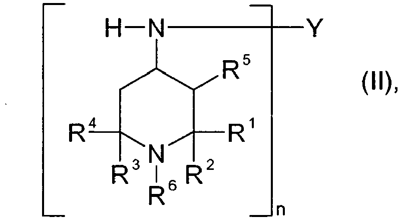 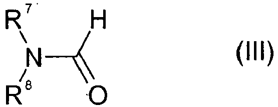 in Gegenwart von Verbindungen der Formel (III)
wobei
R⁷, R⁸ unabhängig voneinander, gleich oder verschieden, Wasserstoff,
C₁-C₂₂-Alkyl, C₃-C₂₂-Alkenyl, unsubstituiertes oder substituiertes
C₇-C₁₂-Phenylalkyl, oder C₁-C₂₂-Alkanoyl, bedeuten,
umgesetzt werden und die Umsetzung in Gegenwart mindestens einer Protonensäure und mindestens einer Lewissäure stattfindet.

Ausdrücke der Form Cₐ-C_{b} bezeichnen im Rahmen dieser Erfindung chemische Verbindungen oder Substituenten mit einer bestimmten Anzahl von Kohlenstoffatomen. Die Anzahl an Kohlenstoffatomen kann aus dem gesamten Bereich von a bis b, einschließlich a und b gewählt werden, a ist mindestens 1 und b immer größer als a. Eine weitere Spezifizierung der chemischen Verbindungen oder der Substituenten erfolgt durch Ausdrücke der Form Cₐ-C_{b}-V. V steht hierbei für eine chemische Verbindungsklasse oder Substituentenklasse, beispielsweise für Alkylverbindungen oder Alkylsubstituenten.

Halogen steht für Fluor, Chlor, Brom, oder Iod, vorzugsweise für Fluor, Chlor oder Brom, besonders bevorzugt für Fluor oder Chlor und speziell für Chlor.

Im einzelnen haben die für die verschiedenen Substituenten angegebenen Sammelbegriffe folgende Bedeutung:
Cₐ-C_{b}-Alkyl: geradkettige oder verzweigte Kohlenwasserstoffreste mit von a bis b Kohlenstoffatomen.

C₁-C₄-Alkyl: geradkettige oder verzweigte Kohlenwasserstoffreste mit bis zu 4 Kohlenstoffatomen, beispielsweise C₁-C₂-Alkyl oder C₃-C₄-Alkyl, bevorzugt C₁-C₂-Alkyl, beispielsweise Methyl, Ethyl, insbesondere Methyl, oder C₃-C₄-Alkyl, Propyl, Isopropyl, n-Butyl, sec-Butyl, tert.-Butyl, 1,1-Dimethylethyl.

C₁-C₂₂-Alkyl: geradkettige oder verzweigte Kohlenwasserstoffreste mit bis zu 22 Kohlenstoffatomen, bevorzugt C₄-C₂₂-Alkyl, beispielsweise C₁-C₁₀-Alkyl oder C₁₁-C₂₂-Alkyl, bevorzugt C₁-C₁₀-Alkyl beispielsweise C₁-C₃-Alkyl, wie Methyl, Ethyl, Propyl, Isopropyl, oder C₄-C₆-Alkyl, n-Butyl, sec-Butyl, tert.-Butyl, 1,1-Dimethylethyl, Pentyl, 2-Methylbutyl, 1,1-Dimethylpropyl, 1,2-Dimethylpropyl, 2,2-Dimethylpropyl, 1-Ethylpropyl, Hexyl, 2-Methylpentyl, 3-Methyl-pentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 2-Ethylbutyl, 1,1,2-Trimethylpropyl, 1,2,2-Tri-methylpropyl, 1-Ethyl-1-methylpropyl, 1-Ethyl-2-methylpropyl, oder C₇-C₁₀-Alkyl, wie Heptyl, Octyl, 2-Ethyl-hexyl, 2,4,4-Trimethylpentyl, 1,1,3,3-Tetramethylbutyl, Nonyl oder Decyl sowie deren Isomere.

C₃-C₂₂-Alkenyl: ungesättigte, geradkettige oder verzweigte Kohlenwasserstoffreste mit 3 bis 22 Kohlenstoffatomen und einer Doppelbindung in einer beliebigen Position, beispielsweise C₃-C₁₀-Alkenyl oder C₁₁-C₂₂-Alkenyl, bevorzugt C₃-C₁₀-Alkenyl wie C₃-C₄-Alkenyl, wie 1-Propenyl, 2-Propenyl, 1-Methylethenyl, 1-Bute-nyl, 2-Butenyl, 3-Butenyl, 1-Methyl-1-propenyl, 2-Methyl-1-propenyl, 1-Methyl-2-pro-penyl, 2-Methyl-2-propenyl, oder C₅-C₆-Alkenyl, wie 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl oder 1-Ethyl-2-methyl-2-propenyl, sowie C₇-C₁₀-Alkenyl, wie die Isomere von Heptenyl, Octenyl, Nonenyl oder Decenyl.

C₇-C₁₂-Phenylalkyl: mit einem Phenylrest substituiertes C₁-C₆-Alkyl mit insgesamt 7 bis 12 Kohlenstoffatomen, beispielsweise C₇-C₁₀-Phenylalkyl oder C₁₁-C₁₂-Phenylalkyl, bevorzugt C₇-C₁₀-Phenylalkyl, beispielsweise Benzyl. Substituierte C₇-C₁₂-Phenylalkyle sind zusätzlich am Phenylring substituiert.

C₇-C₂₂-Phenylalkyl oder Diphenylalkyl: mit einem oder zwei Phenylresten substituiertes C₁-C₁₆-Alkyl oder C₁-C₁₀-Alkyl mit insgesamt 7 bis 22 Kohlenstoffatomen, beispielsweise C₇-C₁₀-Phenylalkyl oder C₁₃-C₁₆-Diphenylalkyl oder C₁₁-C₂₂-Phenylalkyl oder C₁₇-C₂₂-Diphenylalkyl. Substituierte C₇-C₂₂-Phenylalkyle oder Diphenylalkyle sind zusätzlich an einem oder beiden der Phenylringe substituiert.

C₁-C₂₂-Alkanoyl: eine geradkettige oder verzweigte Alkylgruppe mit 1 bis 22 Kohlenstoffatomen (wie vorstehend genannt), welche über eine Carbonylgruppe (-CO-) angebunden sind, beispielsweise C₁-C₁₁-Alkanoyl oder C₁₂-C₂₂-Alkanoyl, bevorzugt C₁-C₁₁-Alkanoyl wie C₁-C₅-Alkanoyl, wie Acetyl, n- oder iso-Propionyl, n-, iso-, sec- oder tert.-Butanoyl, n-, iso-, sec- oder tert.-Pentanoyl, oder C₉-C₁₂-Alkanoyl wie n- oder iso-Nonanoyl, oder n-Dodecanoyl.

C₂-C₃-Cyanalkyl: mit einer CN Gruppe substituiertes C₁-C₂-Alkyl mit insgesamt 2 bis 3 Kohlenstoffatomen. Beispielsweise 2-Cyanethyl.

C₁-C₂₂-Hydroxyalkyl: mit einer Hydroxygruppe an beliebiger Stelle substituiertes C₁-C₂₂-Alkyl mit 1 bis 22 Kohlenstoffatomen.

C₂-C₂₂-Aminoalkyl: mit einer Aminogruppe an beliebiger Stelle substituiertes C₁-C₂₂-Alkyl mit 2 bis 22 Kohlenstoffatomen.

C₃-C₁₂-Cycloalkyl: monocyclische, gesättigte Kohlenwasserstoffgruppen mit 3 bis zu 12 Kohlenstoffringgliedern, bevorzugt C₃-C₈-Cycloalkyl wie Cyclopropyl, Cyclobutyl, Cyclopentyl, Cyclohexyl, Cycloheptyl oder Cyclooctyl.

Bicycloalkyl: bicyclische, gesättigte oder ungesättigte Kohlenwasserstoffsysteme wie zum Beispiel Norbornyl oder Norbenyl.

C₁-C₂₂-Alkoxy: bedeutet eine geradkettige oder verzweigte Alkylgruppe mit 1 bis 22 Kohlenstoffatomen (wie vorstehend genannt), welche über ein Sauerstoffatom (-O-) an gebunden sind, beispielsweise C₁-C₁₀-Alkoxy oder C₁₁-C₂₂-Alkoxy, bevorzugt C₁-C₁₀-Alkyloxy, insbesondere bevorzugt C₁-C₄-Alkoxy, wie beispielweise Methoxy, Ethoxy, Propoxy, Butoxy.

Cₐ-C_{b}-Alkylen: geradkettige oder verzweigte Kohlenwasserstoffreste mit a bis b Kohlenstoffatomen.

C₂-C₂₂-Alkylen: geradkettige oder verzweigte Kohlenwasserstoffreste mit 2 bis 22 Kohlenstoffatomen, beispielsweise C₂-C₁₀-Alkylen oder C₁₁-C₂₂-Alkylen, bevorzugt C₂-C₁₀-Alkylen, insbesondere Tetramethylen, Pentamethylen oder Hexamethylen.

C₅-C₂₂-Cycloalkylen: geradkettige oder verzweigte Kohlenwasserstoffreste mit 5 bis zu 22 Kohlenstoffatomen, enthaltend monocyclische, gesättigte Kohlenwasserstoffgruppen mit 3 bis zu 12 Kohlenstoffringgliedern, bevorzugt 5 bis 8 Kohlenstoffringgliedern.

C₈-C₁₄-Phenylalkylen: geradkettige oder verzweigte Kohlenwasserstoffreste mit 8 bis zu 14 Kohlenstoffatomen, enthaltend eine Phenylengruppe, wobei die Kohlenwasserstoffgruppen außer der Phenylengruppe gesättigt sind.

Heterocyclen: fünf- bis zwölfgliedrige, bevorzugt fünf- bis neungliedrige, besonders bevorzugt fünf- bis sechsgliedrige, Sauerstoff-, Stickstoff- und/oder Schwefelatome, gegebenenfalls mehrere Ringe aufweisende Ringsysteme wie Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert.-Butylthiophenyl. Beispielsweise auch , Piperidinyl, Pyrrolidinyl.

5- oder 6-gliedrige Heterocyclen: 5- oder 6-gliedrige, Sauerstoff-, Stickstoff- und/oder Schwefelatome aufweisende Ringsysteme wie Furyl, Thiophenyl, Pyrryl, Pyridyl, Piperidinyl, Pyrrolidinyl.

Im Rahmen des erfindungsgemäßen Verfahrens erfolgt die Herstellung von 4-Formylaminopiperidinderivaten der Formel (I), sowie der Säureadditionssalze dieser Verbindungen, durch Umsetzung von 4-Aminopiperidinderivaten der Formel (II) in Gegenwart von Verbindungen der Formel (III), bevorzugt in der Gegenwart von Dimethylformamid oder Formamid, insbesondere Formamid. 4-Aminopiperidinderivate der Formel(II) und 4-Formylaminopiperidinderivate der Formel (I) können im erfindungsgemäßen Verfahren als Säureadditionssalze der jeweiligen Verbindung vorliegen beziehungsweise hergestellt werden. Natürlich lassen sich mit Hilfe des erfindungsgemäßen Verfahrens auch Gemische von 4-Formylaminopiperidinderivaten der Formel (I) durch Umsetzung von Gemischen aus 4-Aminopiperidinderivaten der Formel (II) in Gegenwart von Verbindungen der Formel (III) erhalten. Bevorzugt werden im erfindungsgemäßen Verfahren als Verbindungen der Formel (III) reines Formamid oder eine Formamidlösung in einem inerten Lösungsmittel eingesetzt. Ganz bevorzugt setzt man reines Formamid ein. Natürlich lässt sich das erfindungsgemäßen Verfahrens auch mit anderen Einsatzformen der Verbindungen der Formel (III) oder des Formamids durchführen, beispielsweise mit Suspensionen oder Dispersionen. Der Begriff Verbindungen der Formel (III) wird stellvertretend für alle Einsatzformen der Verbindungen der Formel (III) verwendet.

Bevorzugt erfolgt die Herstellung von 4-Formylaminopiperidinderivaten der Formel (I) mit n=2 aus 4-Aminopiperidinderivaten der Formel (II) mit n=2. Nebenprodukt der Herstellung ist für n=2 im wesentlichen nur ein geringer Anteil an einfach umgesetzten Monoamid. Mit Hilfe des erfindungsgemäßen Verfahrens lässt sich ein Monoamidanteil von weniger als 2 mol-% bezogen auf den Gesamtumsatz erhalten. Bevorzugt beträgt der Monamidanteil weniger als 1.5 mol-%. Durch das erfindungsgemäße Verfahren sind daher 4-Formylaminopiperidinderivaten der Formel (I) mit n=2 in hoher Reinheit zugänglich.

Bevorzugt sind in den Formel (I) und (II) R¹, R², R³ und R⁴ unabhängig voneinander, gleich oder verschieden, C₁-C₄-Alkyl. Ganz bevorzugt sind R¹, R², R³ und R⁴ Methyl.

Bevorzugt ist R⁵ Wasserstoff.

Falls R⁶ ein substituiertes C₇-C₁₂-Phenylalkyl ist, so handelt es sich bei den Substituenten bevorzugt um C₁-C₄-Alkyl, Halogen, C₁-C₄-Alkoxy, Methylendioxy, Ethylendioxy und/oder Di-C₁-C₄-alkylamino. Bevorzugt steht R⁶ für Wasserstoff, C₁C₂₂-Alkyl oder C₃-C₂₂-Alkenyl. Besonders bevorzugt ist R⁶ Wasserstoff oder C₁-C₂₂-Alkyl und ganz besonders bevorzugt steht R⁶ für Wasserstoff. Überraschenderweise wird gefunden, dass durch das erfindungsgemäße Verfahren, wenn R⁶ für Wasserstoff steht, eine Formylierung im wesentlichen nur an der 4-Amino-Position der 4-Aminopiperidinverbindung erfolgt. Die N-H Gruppe im Piperidinring bleibt im wesentlichen unverändert bestehen.

Falls Y, wenn n = 1 ist, ein substituiertes C₇-C₁₂- Phenyl- oder Diphenylalkyl ist, so handelt es sich bei den Substituenten bevorzugt um C₁-C₄-Alkyl, Halogen, C₁-C₄-Alkoxy, Methylendioxy, Ethylendioxy und/oder Di-C₁-C₄-alkylamino. Falls Y, wenn n = 1 ist, ein substituiertes Phenyl ist, so handelt es sich bei den Substituenten bevorzugt um C₁-C₄-Alkyl oder C₁-C₄-alkoxy.

Ganz besonders bevorzugt stehen im Falle von n=2 in den Formeln (I) und (II) R¹, R², R³, R⁴, R⁶ für Methyl, R⁵ für Wasserstoff und Y für C6-Alkylen. Die Herstellung der 4-Aminopiperidinderivate der Formel (II) erfolgt in diesem bevorzugten Fall durch dem Fachmann wohlbekannte Verfahren, die beispielsweise in der US 4,331,586, EP 508 940, EP 302020, JP 07304737 und US 4,605,743 beschrieben sind. Beispielsweise erfolgt diese Herstellung durch reduktive Alkylierung von Hexamethylendiamin in Gegenwart von 2,2,6,6-Tetramethyl-4-piperidon unter Einsatz von Hydrogenierungskatalysatoren wie Pd, Pt, Ni, Co in Gegenwart von Wasserstoff entweder in Substanz oder mit Hilfe von Lösungsmitteln. Analog lassen sich viele der 4-Aminopiperidinderivaten der Formel (II) aus den entsprechenden Aminen in Verbindung mit den 4-Piperidonen erhalten. Weitere Herstellungsmethoden der 4-Aminopiperidinderivate der Formel (II) sind dem Fachmann aus dem Stand der Technik wohl bekannt.

Die Umsetzung von 4-Aminopiperidinderivaten der Formel (II) im Rahmen des erfindungsgemäßen Verfahrens kann in Gegenwart eines Lösungsmittels oder "in Substanz" stattfinden. Die Umsetzung in Substanz bedeutet, dass im wesentlichen im erfindungsgemäßen Verfahren keine Lösungsmittel zugegen sind.

Der Begriff "Lösungsmittel" wird im Rahmen dieser Erfindung stellvertretend auch für Verdünnungsmittel gebraucht. Gelöste Stoffe liegen im Lösungsmittel entweder gelöst, suspendiert, in dispergierter oder in emulgierter Form vor. Der Begriff Lösungsmittel umfasst auch Lösungsmittelgemische. Lösungsmittel sind unter den Reaktionsbedingungen des erfindungsgemäßen Verfahrens flüssig. Der Begriff Lösungsmittel umfasst nicht Verbindungen der Formel (III).

Die Umsetzung in Substanz kann bevorzugt dann erfolgen, wenn 4-Aminopiperidinderivate der Formel (II) unter den Reaktionsbedingungen des erfindungsgemäßen Verfahrens in Verbindungen der Formel (III) löslich, suspendierbar, dispergierbar oder emulgierbar sind. Erfindungsgemäß kann die Umsetzung in Substanz auch dann erfolgen, wenn 4-Aminopiperidinderivate der Formel (II) unter den Reaktionsbedingungen des erfindungsgemäßen Verfahrens flüssig sind.

Die Menge an Verbindungen der Formel (III), die bei der Umsetzung in Substanz eingesetzt wird kann, beispielsweise auf Grund der Löslichkeit der 4-Aminopiperidinderivate der Formel (II) in Verbindungen der Formel (III), über einen großen Bereich variieren. Im allgemeinen werden Verbindungen der Formel (III) in von äquimolaren bis fünfzehnfachen molaren Überschuss bezogen auf das 4-Aminopiperidinderivat der Formel (II) zum Einsatz. Bevorzugt wird im Rahmen des erfindungsgemäßen Verfahren eine von äqimolare bis fünffacher molare Menge angewendet. Die Umsetzung in Substanz erfolgt bevorzugt in einem Temperaturbereich von 80 °C bis 180 °C. Besonders bevorzugt erfolgt die Umsetzung in einem Bereich von 100 °C bis 160 °C. Die Umsetzung kann bei Unterdruck, Normaldruck oder unter Überdruck durchgeführt werden. Bevorzugt wird die Reaktion bei Normaldruck oder leichtem Überdruck durchgeführt.

In einer Ausführungsform des erfindungsgemäßen Verfahrens wird ein Lösungsmittel, insbesondere ein inertes Lösungsmittel, verwendet. Inerte Lösungsmittel sind unter den Reaktionsbedingungen des erfindungsgemäßen Verfahrens stabil. Das heißt, die inerten Lösungsmittel zersetzen sich nicht, reagieren nicht mit sich selbst oder Edukten oder Produkten der Umsetzung. Insbesondere sind inerte Lösungsmittel stabil gegenüber Verbindungen der Formel (III), Aminen, oder Ammoniak. Bevorzugt kommen als Lösungsmittel aromatische organische Kohlenwasserstoffe, beispielsweise Toluol, Xylol, Dichlorbenzol, Solvesso™ 100, 150 oder 200 (substituierte Aromatengemische) zum Einsatz. Ganz bevorzugt wird als Lösungsmittel Xylol oder Toluol eingesetzt. Die Menge an eingesetztem Lösungsmittel ist von der Löslichkeit der Einsatzstoffe abhängig und kann in einem weiten Bereich variieren. Durch geeignete Wahl der Menge des Lösungsmittels im erfindungsgemäßen Verfahren werden einerseits unerwünschten Nebeneffekte wie ungenügende Löslichkeit von 4-Aminopiperdinderivaten der Formel (II) beim Einsatz von zuwenig Lösungsmittel, andererseits ein stark erhöhter Energieaufwand beispielsweise für Kühl- oder Heizvorgänge beim Einsatz von zuviel Lösungsmittel vermieden. Im allgemeinen beträgt die Menge an Lösungsmittel von 10 bis 700 Gew.-% bezogen auf die Menge an 4-Aminopiperidinderivat der Formel (II). Bevorzugt werden von 10 bis 500 Gew.-% Lösungsmittel eingesetzt. Ganz bevorzugt kommen von 20 bis 400 Gew.-% Lösungsmittel zum Einsatz.

Die Wahl der Reaktionstemperatur hängt davon ab ob das erfindungsgemäße Verfahren in Substanz oder unter Verwendung eines Lösungsmittels stattfindet. In einer Ausführungsform ist die Reaktionstemperatur abhängig vom Siedepunkt des Lösungsmittels oder vom Siedebereich des Lösungsmittelgemisches. Im erfindungsgemäßen Verfahren werden im allgemeinen Lösungsmittel mit einem Siedepunkt niedriger als der Siedepunkt des 4-Formylaminopiperidinderivats der Formel (I) gewählt. Die Umsetzung unter Verwendung eines Lösungsmittels erfolgt im allgemeinen in einem Temperaturbereich von 100 °C bis 180 °C. Bevorzugt erfolgt die Umsetzung in einem Bereich von 120 °C bis 160 °C. Die Umsetzung kann bei Unterdruck, Normaldruck oder unter leichtem Überdruck durchgeführt werden.

Die Dauer des erfindungsgemäßen Verfahrens bis zur Erreichung eines im wesentlichen vollständigen Umsatzes kann in einem weiten Bereich abhängig von den jeweiligen Einsatzstoffen und den Reaktionsbedingungen variieren. Die Dauer des erfindungsgemäßen Verfahrens beträgt beispielsweise von 1 bis 48 Stunden. Bevorzugt beträgt die Dauer des erfindungsgemäßen Verfahrens weniger als 24 Stunden.

Im allgemeinen lassen sich beliebige Protonen- oder Lewissäuren im erfindungsgemäßen Verfahren einsetzen. Als Protonensäuren werden organische Carbonsäuren, wie Essigsäure oder Propionsäure oder Mineralsäuren wie Salzsäure, Salpetersäure oder Schwefelsäure eingesetzt. Bevorzugt sind wenig korrosive Protonensäuren wie beispielsweise Essigsäure. Als Lewis-Säuren werden Borsäure oder Zinksalze wie Zinkacetat oder Zinkchlorid eingesetzt. Bevorzugt kommt als Lewis-Säure Borsäure zum Einsatz. Die Konzentration der Protonen- oder Lewissäure kann in weiten Bereichen variieren. Bevorzugt beträgt die Konzentration der Protonensäure von 4 Gew.-% bis 40 Gew.-% bezogen auf 4-Aminopiperidinderivat der Formel (II). Besonders bevorzugt beträgt der Konzentrationsbereich von 4 Gew.-% bis 20 Gew.-%. Ganz besonders bevorzugt von 5 Gew.-% bis 10 Gew.-%. Die Konzentration der Lewissäure beträgt bevorzugt von 0.1 Gew.-% bis 2.5 Gew.-% bezogen auf 4-Aminopiperidinderivat der Formel (II). Besonders bevorzugt beträgt der Konzentrationsbereich von 0.2 Gew.-% bis 1.2 Gew.-%. Ganz besonders bevorzugt von 0.3 Gew.-% bis 1.0 Gew.-%.

In einer bevorzugten Ausführungsform findet das erfindungsgemäße Verfahren in Xylol als Lösungsmittel in Gegenwart von Essigsäure (Eisessig) und Borsäure als Katalysator statt.

In einer Ausführungsform findet die Umsetzung im Rahmen des erfindungsgemäßen Verfahrens bei einem Wassergehalt von weniger als 1 Gew.-%, bezogen auf die 4-Aminopiperidinderivate der Formel (II) statt. Bevorzugt beträgt der Wassergehalt weniger als 0.5 Gew.-%. Ganz bevorzugt wird im wesentlichen wasserfrei gearbeitet. In einer Ausführungsform des erfindungsgemäßen Verfahrens werden die Einsatzstoffe so gereinigt, dass der Wassergehalt insgesamt, bezogen auf die 4-Aminopiperidinderivate der Formel (II), weniger als 1 Gew.-% beträgt. Beispielsweise ist zu beachten, dass Verbindungen der Formel (III) häufig hygroskopisch sind. Überschreitet der Wassergehalt die geforderten Grenzwerte, so werden die Verbindungen der Formel (III) vor dem Einsatz gereinigt. Die Reinigung erfolgt im allgemeinen nach dem Fachmann bekannten Methoden, beispielsweise destillativ. Weiterhin ist der Wassergehalt des Lösungsmittels vor dem Einsatz zu ermitteln und gegebenenfalls ist bei Überschreitung wie im Falle der Verbindungen der Formel (III) zu verfahren. Analog wird mit den weiteren Stoffen wie Protonen- und Lewis-Säuren verfahren. Das erfindungsgemäße Verfahren ermöglicht durch den niedrigen Wassergehalt eine hohe Reinheit der Produkte bei gleichzeitigem hohen Umsatz.

In einer Ausführungsform findet die Umsetzung im Rahmen des erfindungsgemäßen Verfahrens im wesentlichen unter Ausschluß von Sauerstoff statt. Der Ausschluß von Sauerstoff wird durch im allgemeinen dem Fachmann bekannte Verfahren erreicht. Beispielsweise wird die Apparatur in der das erfindungsgemäße Verfahren durchgeführt wird, vor Beginn der Umsetzung mit einem inerten Gas, beispielsweise Stickstoff gespült um Sauerstoff zu verdrängen. Die Umsetzung findet bevorzugt unter leichtem Stickstoffüberdruck statt. Beispielsweise beträgt der Stickstoffüberdruck von 5 bis 50 mbar. Bevorzugt beträgt der Stickstoffüberdruck von 10 bis 40 mbar.

In einer anderen Ausführungsform besteht das erfindungsgemäße Verfahren zur Herstellung von 4-Formylaminopiperidinderivaten der Formel (I) im allgemeinen aus einem oder mehreren Verfahrensschritten die zeitlich nacheinander oder auch gleichzeitig ablaufen können. Beispielsweise umfasst das erfindungsgemäße Verfahren die folgenden Verfahrensschritte: Synthese von 4-Formylaminopiperidinderivaten der Formel (I) durch Umsetzung von 4-Aminopiperidinderivaten der Formel (II) in Gegenwart von Verbindungen der Formel (III), optional Entfernung der überschüssigen Verbindungen der Formel (III), optional die Abtrennung eines Lösungsmittels und eines 4-Formylaminopiperidinderivates der Formel (I), optional die Isolierung eines 4-Formylaminopiperidinderivates der Formel (I), optional die Aufarbeitung eines 4-Formylaminopiperidinderivates der Formel (I). Bevorzugt erfolgen diese Schritte zeitlich nacheinander in der Reihenfolge: Synthese, optionale Entfernung, optionale Abtrennung, optionale Isolierung, und optionale Aufarbeitung.

Nach der Beendigung der Umsetzung, d.h. der im wesentlichen vollständigen Beendigung der Synthese im Rahmen des erfindungsgemäßen Verfahrens, können, wenn vorhanden, überschüssige Verbindungen der Formel (III) entfernt werden werden.

In einer bevorzugten Ausführungsform des erfindungsgemäßen Verfahrens werden, wenn vorhanden, überschüssige Verbindungen der Formel (III) desaktiviert. Die optionale Desaktivierung erfolgt im Allgemeinen durch Hydrolyse. Die Hydrolyse kann beispielsweise mit Hilfe von Wasser und/oder wässriger Laugen oder wässrigen Säuren erfolgen. Bevorzugt erfolgt die Hydrolyse mit wässrigen Alkali- oder Erdalkalilaugen. Ganz besonders bevorzugt wird die Hydrolyse in Gegenwart von wässriger Natronlauge durchgeführt.

In einer weiteren bevorzugten Ausführungsform des erfindungsgemäßen Verfahrens werden, wenn vorhanden, überschüssige Verbindungen der Formel (III) destillativ entfernt.

Die Entfernung der überschüssigen Verbindungen der Formel (III), beispielsweise durch Desaktivierung oder Destillation, kann jedoch als Verfahrensschritt vollständig entfallen.

In einer Ausführungsform des erfindungsgemäßen Verfahrens wird nach der optionalen Desaktivierung der überschüssigen Verbindungen der Formel (III) das Lösungsmittel, so vorhanden, abgetrennt. Die Abtrennung des Lösungsmittels erfolgt im allgemeinen auf dem Fachmann wohl bekannte Art und Weise. Bevorzugt kann die Abtrennung destillativ erfolgen. Ganz bevorzugt erfolgt die Abtrennung des Lösungsmittels durch azeotrope Wasserdampf-Destillation. Das Lösungsmittel kann nach Aufbereitung wieder eingesetzt werden. Bevorzugt wird vor der Wiederverwertung des Lösungsmittels Restwasser abgetrennt.

In einer Ausführungsform wird das Lösungsmittel, insbesondere Xylol, nach erfolgter Umsetzung und Hydrolyse von überschüssigen Verbindungen der Formel (III), optional nach Zugabe von Wasser, azeotrop abdestilliert und wiederverwendet. Bevorzugt wird vor der Wiederverwertung des Lösungsmittels, insbesondere des Xylols, Restwasser ausgekreist.

Nach der optionalen Desaktivierung der überschüssigen Verbindungen der Formel (III) und der optionalen Abtrennung des Lösungsmittels erfolgt im Rahmen des erfindungsgemäßen Verfahrens eine optionale Isolierung der 4-Formylaminopiperidinderivate der Formel (I) vom übrigen Reaktionsgemisch. Der Begriff Reaktionsgemisch umfasst die während der einzelnen Verfahrensschritte des erfindungsgemäßen Verfahrens auftretenden Stoffgemische. Beispielsweise bezeichnet Reaktionsgemisch eine Mischung aus Edukten, Produkten, Lösungsmittel, und Katalysatoren. Insbesondere umfasst der Begriff Reaktionsgemisch auch im erfindungsgemäßen Verfahren ausgefallene Feststoffe im Kontakt mit dem Lösungsmittel und dem noch im Lösungsmittel gelösten Verbindungen.

In einer bevorzugten Ausführungsform des erfindungsgemäßen Verfahrens wird bei der Isolierung das 4-Formylaminopiperidinderivat der Formel (I) destillativ aus dem Reaktionsgemisch entfernt.

In einer anderen bevorzugten Ausführungsform des erfindungsgemäßen Verfahrens trennt man das 4-Formylaminopiperidinderivaten der Formel (I) auf Grund mangelnder Löslichkeit von einer wässrigen Phase ab. Eine wässrige Phase entsteht bei der Durchführung des erfindungsgemäßen Verfahrens beispielsweise durch die Hydrolyse von Verbindungen der Formel (III) mit Hilfe wässriger Laugen und optional auch bei der Abtrennung des Lösungsmittels durch azeotrope Wasserdampf-Destillation. Die wässrige Phase lässt sich jedoch auch auf andere Art und Weise herstellen, beispielsweise durch Zugabe von Wasser oder im wesentlichen wässrigen Lösungen zum Reaktionsgemisch. Bevorzugt erfolgt die Isolierung des 4-Formylaminopiperidinderivats der Formel (I) aus der wässrigen Phase durch Kristallisation. Die wässrige Phase wird in diesem Fall als Mutterlauge bezeichnet.

Im Rahmen einiger Ausführungsformen des erfindungsgemäßen Verfahrens lässt sich die Isolierung, beispielsweise die Kristallisation, durch die Verwendung erhöhter Wassermengen in der Hydrolyse oder durch die Zugabe eines Lösungsmittels, beispielsweise Xylol nach der Umsetzung, aber vor Beginn der Desaktivierung, beispielsweise durch Hydrolyse, beschleunigen oder verbessern.

Die weitere optionale Aufarbeitung des 4-Formylaminopiperidinderivats der Formel (I) erfolgt im Rahmen des erfindungsgemäßen Verfahrens im allgemeinen nach dem Fachmann wohl bekannten Techniken. Beispielhaft sei hier eine Abfolge von Filtrations-, Wasch-, und Trocknungsschritten beschrieben. Alternativ hierzu lässt sich beispielsweise eine Abfolge von Zentrifugations-, Wasch-, und Trocknungsschritten ausführen. Bei flüssigen Produkten erfolgt die Isolierung und Aufarbeitung beispielsweise durch gängige, dem Fachmann wohlbekannte Destillationsschritte.

In einer Ausführungsform des erfindungsgemäßen Verfahrens wird das Reaktionsgemisch nach der Umsetzung einer Filtration unterworfen. Die Filtration kann aus einem oder mehreren Filtrationsschritten bestehen und das 4-Formylaminopiperidinderivat der Formel (I) verbleibt im festen Filtrationsrückstand. Um eine höhere Reinheit des 4-Formylaminopiperidinderivats der Formel (I) nach dem erfindungsgemäßen Verfahren zu gewährleisten schließt sich im allgemeinen ein oder mehrere Waschschritt(e) an die Filtration an. Der Filtrationsrückstand wird bevorzugt mit Wasser gewaschen. Isolierung und Aufarbeitung des 4-Formylaminopiperidinderivats der Formel (I) lassen sich jedoch im Rahmen des erfindungsgemäßen Verfahrens auch nach anderen Techniken durchführen.

In einer anderen Ausführungsform des erfindungsgemäßen Verfahrens wird dem Reaktionsgemisch ein flüssiges oder in einem inerten Lösemittel gelöstes, emulgiertes oder suspendiertes Additiv zugegeben.

Die Zugabe des Additivs zum Reaktionsgemisch kann grundsätzlich zu einem beliebigen Zeitpunkt, beispielsweise vor, während und/oder nach der erfindungsgemäßen-Umsetzung von 4-Aminopiperidinderivaten der Formel (II) erfolgen. Bevorzugt erfolgt diese Zugabe während und/oder nach der Umsetzung. Ganz bevorzugt erfolgt die Zugabe eines flüssigen oder in einem inerten Lösemittel gelösten, emulgierten oder suspendierten Additivs nach der Umsetzung.

Bevorzugt wird das Additiv in einer Menge von 0,01 bis 1,5 Gew.-%, besonders bevorzugt von 0,01 bis 0,75 Gew.-%, ganz besonders bevorzugt von 0,01 bis 0,25 Gew.-% zugesetzt. Bei dem Additiv handelt es sich bevorzugt um ein Additiv aus der Klasse der Kunststoff-Additive, wie sie z.B. im "Plastics Additives Handbook", Verlag Carl Hanser, ISBN 978-3-446-19579-0, beschrieben sind. Besonders bevorzugt handelt es sich bei dem Kunststoff-Additiv um eine Verbindung aus der Klasse der Antioxidantien, Metalldesaktivatoren, weiterer Lichtschutzmittel, Weichmacher, Gleitmittel, Flammschutzmittel, Antistatika , optischen Aufheller oder Farbmittel. Ganz bevorzugt handelt es sich beid dem Kunststoff-Additiv um ein Weißöl, wie z.B. Pionier® 2071 oder 1115 (Fa. Pionier, CAS 8042-47-5), Tudalen® 3036(CAS 64741-89-5) oder Winog® 70 (Fa. H&R, CAS 8012-95-1), Enerpar® M006 oder M002 (Fa. BP, CAS 8042-47-5), Primol® 382 (Exxon Mobile, CAS 8012-95-1), Ondina Öl G17 (Fa. Esso, CAS 8042-47-5).

Das erfindungsgemäße Verfahren kann in beliebigen Apparaturen durchgeführt werden, die die Ausführung der (optionalen) Verfahrensschritte erlauben. Die Apparaturen zur Durchführung beispielsweise: der Synthese von 4-Formylaminopiperidinderivaten der Formel (I) durch Umsetzung von 4-Aminopiperidinderivaten der Formel (II) in Gegenwart von Verbindungen der Formel (III), der optionalen Desaktivierung der überschüssigen Verbindungen der Formel (III), der optionalen Abtrennung eines Lösungsmittels und eines 4-Formylaminopiperidinderivates der Formel (I), der optionalen Isolierung eines 4-Formylaminopiperidinderivates der Formel (I), oder der optionalen Aufarbeitung eines 4-Formylaminopiperidinderivates der Formel (I), sind dem Fachmann im allgemeinen wohl bekannt.

Die erfindungsgemäß hergestellten 4-Formylaminopiperidinderivate lassen sich zum Schutz unbelebter organischer Materie vor der Einwirkung von Licht verwenden, insbesondere als Lichtschutzmittel gegen die schädigende Einwirkung von UV-Strahlung. Beispielsweise werden sie als Lichtschutzmittel für Polymere eingesetzt. Unter Polymeren sind hierbei beliebige Kunststoffe, bevorzugt thermoplastische Kunststoffe, insbesondere Folien, Fasern oder Formkörper beliebiger Gestalt zu verstehen. Die Polymere sind beispielsweise Polyolefine, Polyamide, Polystyrole, Polyacrylnitrile, Polycarbonate, Acrylnitril-Butadien-Styrole (ABS), Polyvinylchloride, Polyurethane, oder Polyester. Polymere können auch Co-Polymere, Mischungen oder Blends der oben genannten Polymere sein. Bevorzugte Polymere sind Polyolefine, insbesondere Polyethylen oder Polypropylen. Weiterhin bevorzugte Polymere sind Polystyrole oder ABS. Ebenfalls bevorzugte Polymere sind Polyamide.

Das erfindungsgemäße Verfahren gestattet die Herstellung von 4-Formylaminopiperidinderivaten. Im Rahmen des erfindungsgemäßen Verfahrens kann der Einsatz korrosiver Substanzen wie Ameisensäure vermieden werden. Das erfindungsgemäße Verfahren zeichnet sich durch eine einfache Reaktionsführung aus mit der 4-Formylaminopiperidinderivate in hoher Reinheit bei hohen Umsätzen zugänglich sind.

Die vorstehenden Ausführungsformen des erfindungsgemäßen Verfahrens und die nachfolgenden Beispiele verdeutlichen beispielhaft die vorliegende Erfindung. Es sind jedoch viele weitere Variationen des Verfahrens und Kombinationen der Merkmale des erfindungsgemäßen Verfahrens für den Fachmann denkbar, ohne den Rahmen der Patentansprüche zu verlassen.

### Beispiele:

Die Reinheit der Produkte wurde mit Hilfe der Gaschromatographie bestimmt.

### Beispiel 1: Herstellung von N,N'-1,6-Hexandiylbis[N-(2,2,6,6-tetramethyl-4-piperidinyl)-formamid mit Lösungsmittel: Xylol

Zu 60 ml Xylol werden 40 ml Formamid, 170 g N,N'-bis(2,2,6,6-tetramethyl-4-piperidinyl)- 1,6-Hexandiamin und 0.9 g Borsäure hinzugefügt. Man gibt 20 ml Essigsäure hinzu und erwärmt unter Inertgasatmosphäre auf Rückflusstemperatur. Nach etwa 10 Stunden Reaktionszeit wird das Reaktionsgemisch auf ungefähr 90 bis 95°C abgekühlt und man lässt unter Rühren 40 ml Wasser zulaufen. Anschließend wird unter weiterem Rühren ausreichend wässrige Natronlauge zur Desaktivierung des überschüssigen Formamids zugesetzt. Das Reaktionsgemisch wird erneut zum Sieden gebracht und Xylol wird azeotrop abdestilliert. Anschließend kühlt man auf ungefähr 60°C ab, fügt nochmals Wasser hinzu, und trennt den entstandenen festen Produktrückstand ab. Der Produktrückstand wird mit Wasser nachgewaschen und dann getrocknet. Nach dem Trocknen erhält man ca. 185 g Produkt (95% Ausbeute) in einer Reinheit von größer 99%. Der Schmelzpunkt des Produktes liegt bei 157-158 °C.

### Beispiel 2: Herstellung von N,N'-1,6-Hexandiylbis[N-(2,2,6,6-tetramethyl-4-piperidinyl)-formamid ohne Lösungsmittel:

Zu 72 g Formamid werden 25 g Essigsäure, 0.5 g Borsäure, 157 g N,N'-Bis(2,2,6,6-tetramethyl-4-piperidinyl)- 1,6-hexandiamin gegeben und etwa 6 Stunden bei ungefähr 160°C gerührt. Unter Rückfluss und Siedekühlung werden 150ml Wasser und ausreichend wässrige Natronlauge zur Desaktivierung des überschüssigen Formamids zugetropft und weitere 2 Stunden bei ungefähr 95°C gerührt, wobei das Produkt fein auskristallisiert. Nach dem Abkühlen auf Raumtemperatur trennt man den Produktrückstand ab und wäscht den Produktrückstand mit Wasser. Man erhält das Produkt in einer Reinheit von 99.6% und mit einer Ausbeute von 89%.

### Beispiel 3: Herstellung von N-Cyclohexyl-N-(2,2,6,6-tetramethyl-piperidin-4-yl)-formamid

19.1 g N-Cyclohexyl-N-2,2,6,6-tetramethyl-piperidin werden mit 50 g Formamid, 0.12 g Borsäure und 1 ml Essigsäure versetzt und unter Inertgasatmosphäre etwa 12 Stunden bei ungefähr 160 °C gerührt. Anschließend fügt man bei ungefähr 100°C 50 ml Wasser und innerhalb einer Stunde ausreichend wässrige Natronlauge zur Desaktivierung des überschüssigen Formamids zu und rührt eine Stunde bei ungefähr 90°C nach. Nach Abkühlen auf Raumtemperatur trennt man den Produktrückstand ab und trocknet ihn. Man erhält 11.5 g des farblosen Produkts mit einer Reinheit von 99.8%

### Beispiel 4: Herstellung von N-Butyl, 2,2,6,6 Tetramethyl-4-piperidinaminformamid

43,4gN-Butyl, 2,2,6,6 Tetramethyl-4-piperidinamin,100 ml Xylol, 18g Formamid, 1g Borsäure und 10g Essigsäure werden etwa 6 Stunden unter Inertgasatmosphäre bei Rückfluss gerührt. Nach Abkühlen auf Raumtemperatur werden zwei Phasen erhalten, die im Vakuum destilliert werden. Das Produkt geht bei 180-182°C/21 mbar über. Man erhält 45 g (92%) einer klaren Flüssigkeit mit einer Reinheit von 99.5%.

### Beispiel 5: Zu 72 g Formamid werden 25 g Essigsäure, 0.5 g Borsäure, 157 g N,N'-

Bis(2,2,6,6-tetramethyl-4-piperidinyl)- 1,6-hexandiamin gegeben und 8.5 Stunden bei ungefähr 160°C gerührt. Anschließend werden 0.3 Gew.%, bezogen auf das Produkt, mineralisches Weißöl Pionier® 2071 zugegeben. Unter Rückfluss und Siedekühlung werden 150ml Wasser und ausreichend wässrige Natronlauge zur Desaktivierung des überschüssigen Formamids zugetropft und weitere 2 Stunden bei ungefähr 95°C gerührt, wobei das Produkt fein auskristallisiert. Nach dem Abkühlen auf Raumtemperatur trennt man den Produktrückstand ab und wäscht den Produktrückstand mit Wasser. Man erhält das Produkt in einer Reinheit von 99.6% und mit einer Ausbeute von 96.5%.

## Patentansprüche

1. Verfahren zur Herstellung von 4-Formylaminopiperidinderivaten der Formel (I) 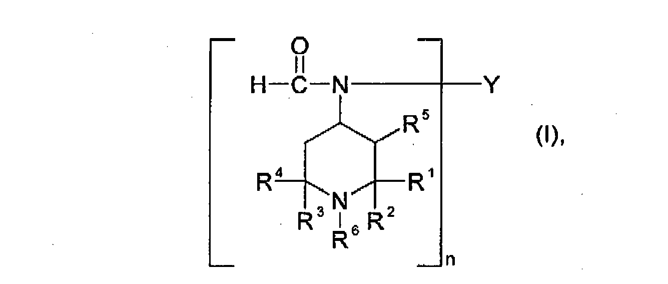 in der
n 1 oder 2,
R¹, R², R³ und R⁴ unabhängig voneinander, gleich oder verschieden, C₁-C₄-Alkyl oder R¹ und R² oder R³ und R⁴ zusammen eine Tetramethylen- oder Pentamethylengruppe,
R⁵ Wasserstoff oder C₁-C₄-Alkyl,
R⁶ Wasserstoff, Sauerstoff, C₁-C₂₂-Alkyl, C₃-C₂₂-Alkenyl, C₁-C₂₂-Alkoxy, unsubstituiertes oder substituiertes C₇-C₁₂-Phenylalkyl, C₁-C₂₂-Alkanoyl, C₂-C₃-Cyanalkyl, C₁-C₂₂-Hydroxyalkyl oder C₂-C₂₂-Aminoalkyl und
- wenn n = 1 ist -
Y Wasserstoff, C₁-C₂₂-Alkyl, C₃-C₂₂-Alkenyl, C₃-C₁₂-Cycloalkyl oder Bicycloalkyl, durch Cyan, Hydroxy oder C₁-C₄-alkoxy substituiertes C₂-C₂₂-Alkyl, durch Ethersauerstoff, Stickstoff oder Schwefel unterbrochenes C₄-C₂₂-Alkyl, unsubstituiertes oder substituiertes C₇-C₂₂-Phenyl- oder Diphenylalkyl, unsubstituiertes oder substituiertes Phenyl, ein Rest der Formel
oder heterocyclische Reste enthaltendes C₁-C₂₂-Alkyl, oder 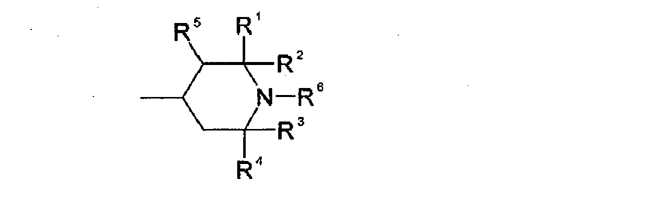 - wenn n = 2 ist -
Y C₂-C₂₂-Alkylen, C₅-C₂₂-Cycloalkylen, C₈-C₁₄-Phonylalkylen, Phenylen oder durch Ethersauerstoff, Stickstoff, Schwefel oder 5 - oder 6-gliedrige Heterocyclen unterbrochenes C₄-C₃₀-Alkylen
bedeuten,
sowie den Säureadditionssalzen dieser Verbindungen,
umfassend die Umsetzung von 4-Aminopiperidinderivaten der Formel (II) oder der Säureadditionssalze dieser Verbindungen 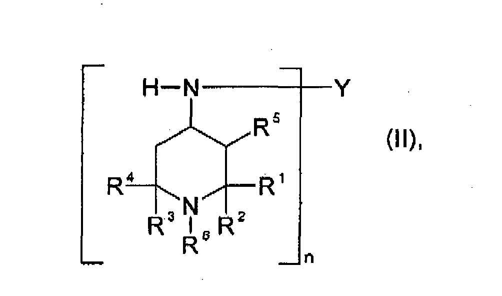
in Gegenwart von Verbindungen der Formel (III) 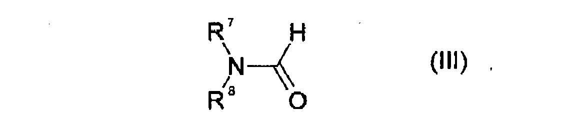 wobei
R⁷, R⁸ unabhängig voneinander, gleich oder verschieden, Wasserstoff, C₁-C₂₂-Alkyl, C₃-C₂₂-Alkenyl, unsubstituiertes oder substituiertes C₇-C₁₂-Phenylalkyl, oder C₁-C₂₂-Alkanoyl, bedeuten,
und
die Umsetzung in Gegenwart mindestens einer Protonensäure und mindestens einer Lewissäure stattfindet.

2. Verfahren nach Anspruch 1, **dadurch gekennzeichnet, dass** die Umsetzung in Gegenwart eines gegenüber Verbindungen der Formel (III), Aminen, oder Ammoniak inerten Lösungsmittels stattfindet.

3. Verfahren nach Anspruch 1, **dadurch gekennzeichnet, dass** die Umsetzung ohne die Gegenwart eines Lösungsmittel stattfindet.

4. Verfahren nach Anspruch 2, **dadurch gekennzeichnet, dass** als Lösungsmittel ein aromatischer organischer Kohlenwasserstoff eingesetzt wird.

5. Verfahren nach den Ansprüchen 1 bis 4, **dadurch gekennzeichnet, dass** während der Umsetzung Sauerstoff im wesentlichen ausgeschlossen bleibt.

6. Verfahren nach den Ansprüchen 1 bis 5, **dadurch gekennzeichnet, dass** während der Umsetzung im wesentlichen wasserfrei gearbeitet wird.

7. Verfahren nach den Ansprüchen 1 bis 6, wobei man nach Beendigung der Umsetzung der 4-Aminopiperidinderivaten der Formel (II) gemäß Anspruch 1 überschüssige Verbindungen der Formel (III) desaktiviert.

8. Verfahren nach den Ansprüchen 1 bis 6, wobei man nach Beendigung der Umsetzung der 4-Aminopiperidinderivaten der Formel (II) gemäß Anspruch 1 überschüssige Verbindungen der Formel (III) destillativ entfernt.

9. Verfahren zur Abtrennung von gemäß einem der Ansprüche 2 oder 5 bis 8 hergestellten 4-Formylaminopiperidinderivat der Formel (I), wobei man das Lösungsmittel nach Zugabe von Wasser azeotrop abdestilliert.

10. Verfahren nach den Ansprüchen 1 bis 9, wobei man der Reaktionsmischung zusätzlich ein flüssiges oder in einem gemäß Anspruch 2 inerten Lösemittel gelöstes, emulgiertes oder suspendiertes Additiv zusetzt.

11. Verfahren nach Anspruch 10, wobei es sich bei dem Additiv um ein Additv aus der Klasse der Kunststoff-Additive handelt.

12. Verfahren nach Anspruch 11, wobei es sich bei dem Kunststoff-Additiv um eine Verbindung aus der Klasse der Antioxidantien, Metalldesaktivatoren, Lichtschutzmittel, Weichmacher, Gleitmittel, Flammschutzmittel, Antistatika, optischen Aufheller oder Farbmittel handelt.

13. Verfahren nach den Ansprüchen 1 bis 12, wobei die Umsetzung in Gegenwart von Essigsäure und Borsäure stattfindet.

## Claims

1. A method for producing 4-formylaminopiperidine derivatives of the formula (I) 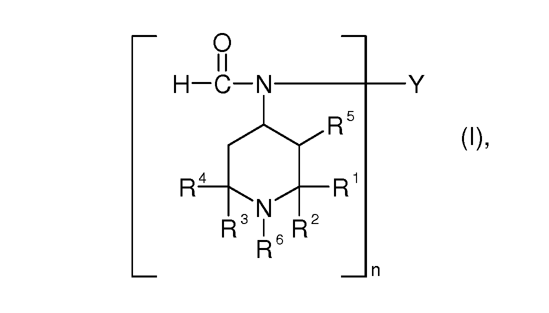 in which
n is 1 or 2,
R¹, R², R³ and R⁴, independently of one another, are identical or different and are C₁-C₄-alkyl or R¹ and R² or R³ and R⁴ together are a tetramethylene or pentamethylene group,
R⁵ is hydrogen or C₁-C₄-alkyl,
R⁶ is hydrogen, oxygen, C₁-C₂₂-alkyl, C₃-C₂₂-alkenyl, C₁-C₂₂-alkoxy, unsubstituted or substituted C₇-C₁₂-phenylalkyl, C₁-C₂₂-alkanoyl, C₂-C₃-cyanoalkyl, C₁-C₂₂-hydroxyalkyl or C₂-C₂₂-aminoalkyl and
- if n = 1 -
Y is hydrogen, C₁-C₂₂-alkyl, C₃-C₂₂-alkenyl, C₃-C₁₂-cycloalkyl or bicycloalkyl, C₂-C₂₂-alkyl substituted by cyano, hydroxy or C₁-C₄-alkoxy, C₄-C₂₂-alkyl interrupted by ether oxygen, nitrogen or sulfur, unsubstituted or substituted C₇-C₂₂-phenyl or diphenylalkyl, unsubstituted or substituted phenyl, a radical of the formula
or C₁-C₂₂-alkyl comprising heterocyclic radicals, or 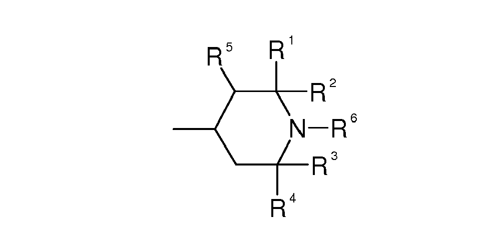 - when n = 2 -
Y is C₂-C₂₂-alkylene, C₅-C₂₂-cycloalkylene, C₈-C₁₄-phenylalkylene, phenylene or C₄-C₃₀-alkylene interrupted by ether oxygen, nitrogen, sulfur or 5- or 6-membered heterocycles,
and also the acid addition salts of these compounds,
comprising the reaction of 4-aminopiperidine derivatives of the formula (II) or the acid addition salts of these compounds 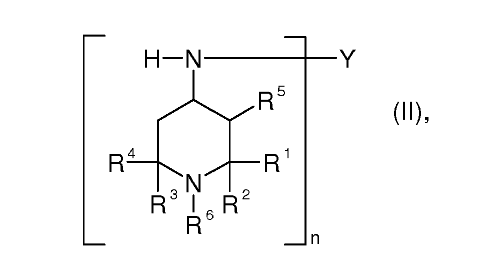
in the presence of compounds of the formula (III) 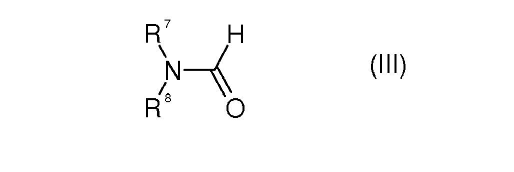 where
R⁷, R⁸, independently of one another, are identical or different and are hydrogen, C₁-C₂₂-alkyl, C₃*-*C₂₂-alkenyl, unsubstituted or substituted C₇-C₁₂-phenylalkyl, or C₁-C₂₂-alkanoyl,
and
the reaction takes place in the presence of at least one protic acid and at least one Lewis acid.

2. The method according to claim 1, wherein the reaction takes place in the presence of a solvent inert toward compounds of the formula (III), amines, or ammonia.

3. The method according to claim 1, wherein the reaction takes place without the presence of a solvent.

4. The method according to claim 2, wherein the solvent used is an aromatic organic hydrocarbon.

5. The method according to claims 1 to 4, wherein oxygen remains essentially excluded during the reaction.

6. The method according to claims 1 to 5, wherein processing is essentially water-free during the reaction.

7. The method according to claims 1 to 6, where, after the reaction of the 4-aminopiperidine derivatives of the formula (II) according to claim 1 is complete, excess compounds of the formula (III) are deactivated.

8. The method according to claims 1 to 6, where, after the reaction of the 4-aminopiperidine derivatives of the formula (II) according to claim 1 is complete, excess compounds of the formula (III) are removed by distillation.

9. A method for separating off 4-formylaminopiperidine derivative of the formula (I) produced according to one of claims 2 or 5 to 8, where, following the addition of water, the solvent is distilled off azeotropically.

10. The method according to claims 1 to 9, where additionally a liquid additive or an additive suspended, emulsified or dissolved in a solvent inert according to claim 2 is added to the reaction mixture.

11. The method according to claim 10, where the additive is an additive from the class of plastics additives.

12. The method according to claim 11, where the plastics additive is a compound from the class of antioxidants, metal deactivators, photoprotective agents, plasticizers, lubricants, flame retardants, antistats, optical brighteners or colorants.

13. The method according to claims 1 to 12, wherein the reaction takes place in the presence of acetic acid and boric acid.

## Revendications

1. Procédé de fabrication de dérivés de 4-formylaminopipéridine de formule (I) 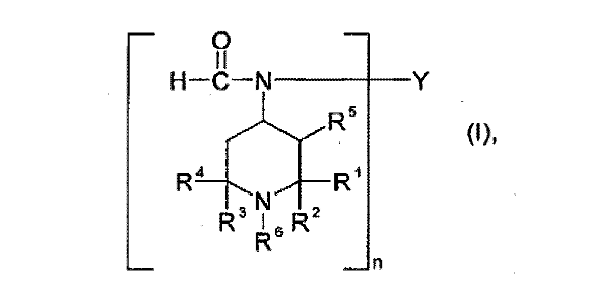 dans laquelle
n signifie 1 ou 2,
R¹, R², R³ et R⁴ signifient indépendamment les uns des autres, de manière identique ou différente, alkyle en C₁-C₄, ou R¹ et R² ou R³ et R⁴ signifient ensemble un groupe tétraméthylène ou pentaméthylène,
R⁵ signifie hydrogène ou alkyle en C₁-C₄,
R⁶ signifie hydrogène, oxygène, alkyle en C₁-C₂₂, alcényle en C₃*-*C₂₂, alcoxy en C₁-C₂₂, phénylalkyle en C₇-C₁₂ non substitué ou substitué, alcanoyle en C₁-C₂₂, cyanoalkyle en C₂-C₃, hydroxyalkyle en C₁-C₂₂ ou aminoalkyle en C₂-C₂₂, et
- lorsque n = 1,
Y signifie hydrogène, alkyle en C₁-C₂₂, alcényle en C₃-C₂₂, cycloalkyle en C₃-C₁₂ ou bicycloalkyle, alkyle en C₂-C₂₂ substitué par cyano, hydroxy ou alcoxy en C₁-C₄, alkyle en C₄-C₂₂ interrompu par oxygène éthéré, azote ou soufre, phényl- ou diphénylalkyle en C₇-C₂₂ non substitué ou substitué, phényle non substitué ou substitué, un radical de formule
ou des radicaux hétérocycliques contenant alkyle en C₁-C₂₂, ou 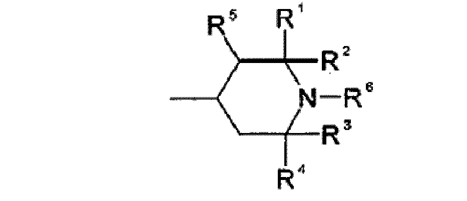 - lorsque n = 2,
Y signifie alkylène en C₂-C₂₂, cycloalkylène en C₅-C₂₂, phénylalkylène en C₈-C₁₄, phénylène ou alkylène en C₄-C₃₀ interrompu par oxygène éthéré, azote, soufre ou hétérocycle à 5 ou 6 éléments,
ainsi que les sels d'addition acide de ces composés, comprenant la mise en réaction de dérivés de 4-aminopipéridine de formule (II) ou des sels d'addition acide de ces composés 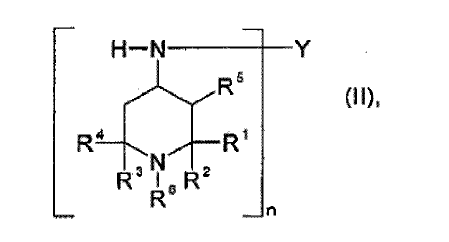
en présence de composés de formule (III) 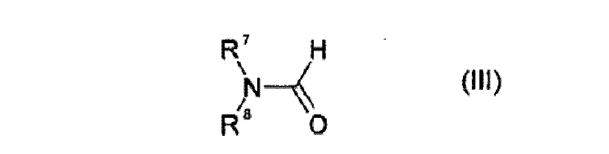 dans laquelle
R⁷, R⁸ signifient indépendamment l'un de l'autre, de manière identique ou différente, hydrogène, alkyle en C₁-C₂₂, alcényle en C₃-C₂₂, phénylalkyle en C₇-C₁₂ non substitué ou substitué, ou alcanoyle en C₁-C₂₂,
la mise en réaction ayant lieu en présence d'au moins un acide protique et d'au moins un acide de Lewis.

2. Procédé selon la revendication 1, **caractérisé en ce que** la mise en réaction a lieu en présence d'un solvant inerte vis-à-vis des composés de formule (III), des amines ou de l'ammoniac.

3. Procédé selon la revendication 1, **caractérisé en ce que** la mise en réaction a lieu sans la présence d'un solvant.

4. Procédé selon la revendication 2, **caractérisé en ce qu'**un hydrocarbure organique aromatique est utilisé en tant que solvant.

5. Procédé selon les revendications 1 à 4, **caractérisé en ce que** l'oxygène reste essentiellement exclu pendant la réaction.

6. Procédé selon les revendications 1 à 5, **caractérisé en ce que** la réaction est réalisée en conditions essentiellement anhydres.

7. Procédé selon les revendications 1 à 6, dans lequel les composés de formule (III) en excès sont désactivés après la fin de la réaction des dérivés de 4-aminopipéridine de formule (II) selon la revendication 1.

8. Procédé selon les revendications 1 à 6, dans lequel les composés de formule (III) en excès sont éliminés par distillation après la fin de la réaction des dérivés de 4-aminopipéridine de formule (II) selon la revendication 1.

9. Procédé de séparation d'un dérivé de 4-formylaminopipéridine de formule (I) fabriqué selon l'une quelconque des revendications 2 ou 5 à 8, dans lequel le solvant est éliminé par distillation azéotropique après ajout d'eau.

10. Procédé selon les revendications 1 à 9, dans lequel un additif liquide ou dissous, émulsifié ou suspendu dans un solvant inerte selon la revendication 2 est en outre ajouté au mélange réactionnel.

11. Procédé selon la revendication 10, dans lequel l'additif est un additif de la classe des additifs pour plastiques.

12. Procédé selon la revendication 11, dans lequel l'additif pour plastiques est un composé de la classe des antioxydants, des désactivateurs de métaux, des agents photoprotecteurs, des plastifiants, des lubrifiants, des agents ignifuges, des antistatiques, des azurants optiques ou des colorants.

13. Procédé selon les revendications 1 à 12, dans lequel la réaction a lieu en présence d'acide acétique et d'acide borique.
